# EUROPÄISCHE PATENTSCHRIFT

(11) **EP 0 636 033 B1**
(45) Veröffentlichungstag und Bekanntmachung des Hinweises auf die Patenterteilung: **07.11.2001**
(21) Anmeldenummer: 92907663.6
(22) Anmeldetag: 11.04.1992
(51) Int. Cl.: A61L 27/00, A61L 27/38

(54) **VERFAHREN ZUR HERSTELLUNG VON WIRKSTOFFKOMPLEXEN**
PROCESS FOR PRODUCING ACTIVE AGENT COMPLEXES
PROCEDE POUR LA FABRICATION DE COMPLEXES DE SUBSTANCES ACTIVES

(43) Veröffentlichungstag der Anmeldung: 01.02.1995
(73) Patentinhaber: SCHMIDT, Karlheinz, Prof. Dr. Dr., 72810 Gomaringen (DE)
(72) Erfinder: SCHMIDT, Karlheinz, Prof. Dr. Dr., 72810 Gomaringen (DE)
(74) Vertreter: Weiss, Ursula, Dr.
(86) Internationale Anmeldenummer: EP9200822
(87) Internationale Veröffentlichungsnummer: WO9320857

(56) Entgegenhaltungen:
- WO-A-91/06324
- US-A- 4 681 763

## Beschreibung

Die Erfindung betrifft ein Verfahren zur Herstellung von Wirkstoffkomplexen mit den Merkmalen des Oberbegriffs des Anspruches 1. Die Erfindung betrifft auch einen nach dem Verfahren hergestellten Wirkstoffkomplex für die Herstellung von biologischen Teilen und seine Verwendung.

Wirkstoffkomplexe sind solche Komplexe, die mindestens zwei Komponenten aufweisen, wobei zumindest eine der Komponenten ein Wirkstoff ist. Wirkstoffe entfalten ihre Wirkungen im allgemeinen dadurch, daß sie mit Zellen als den Trägern der Lebensfunktionen in Wechselwirkung treten. Dabei können die Wirkstoffe endogen, d.h. im Inneren des Körpers als Produkte des zellulären Stoffwechsels hergestellt oder im Sinne von Nahrungs-, Umwelt-oder Arzneistoffen dem Körper von außen zugeführt werden. In allen Fällen ist zur Erzielung der biologischen Wirkung die Wechselwirkung mit einer oder mehreren Zielstrukturen im Organismus erforderlich.

Bei endogen produzierten Wirkstoffen können die räumlichen und zeitlichen Distanzen zwischen ihrer Produktion und Wirkung sehr unterschiedlich sein. So können beispielsweise Hormone über große Distanzen wirken oder aber auch als Gewebshormone ihre Aktivitäten über sehr kurze Strecken entfalten. Um Veränderungen im Signalfluß zwischen Produktionsort und Wirkort eines Wirkstoffes kurzfristig möglich zu machen, ist bei vielen endogen produzierten Wirkstoffen, wie z.B. Hormonen, Neurotransmittern, Cytokinen usw. die Halbwertszeit kurz, was zur Folge hat, daß die Lebenszeit der Wirkstoffe im Ausbreitungsmedium sehr kurz ist. Die Halbwertszeiten liegen beispielsweise im Blut für viele Wirkstoffe im Bereich von Minuten bis Sekunden.

Diese Produktions- und Wirkungscharakteristik mit sehr raschen Abbaukinetiken ist für die endogenen Wirkstoffe meist vorteilhaft, da eine rasche Anpassung der Produktion an den Bedarf möglich ist, stellt jedoch für die exogene Anwendung dieser kurzlebigen Wirkstoffe ein schwerwiegendes Problem dar. Denn in vielen Fällen ist die exogene Anwendung bedeutsamer Wirkstoffe deswegen nicht möglich, weil ihre Halbwertszeiten so kurz sind, daß es nicht mehr zur Wechselwirkung mit den entsprechenden Zielstrukturen kommt und damit die Wirkung ausbleibt.

Bei einem bekannten Verfahren für die Herstellung von Wirkstoffkomplexen wird ein Ausgangskomplex, der als Komponenten unter anderem einen oder mehrere gewünschte Wirkstoffe aufweist, einem Denaturierungsvorgang unterzogen, wobei der Ausgangskomplex zumindest teilweise und unter Zerlegung in seine Komponenten in eine homogene Phase gebracht wird, und bei dem einzelne Komponenten ihre Struktur zumindest teilweise ändern. Bei diesem Denaturierungsvorgang wird darüber hinaus der jeweilige Ausgangskomplex physiologisch inaktiviert. Jeder gewünschte Wirkstoff wird dann aus der homogenen Phase isoliert und für den Erhalt des Wirkstoffkomplexes entsprechend "gesammelt". Wirkstoffkomplexe, die nach diesem bekannten Verfahren hergestellt sind, sind nicht dafür geeignet, kurzlebige Wirkstoffe in einem lebenden Organismus zum Einsatz zu bringen.

Der Erfindung liegt daher die Aufgabe zugrunde, ein Verfahren zum Herstellen von Wirkstoffkomplexen zu schaffen, deren Wirkung sich auch bei Verwendung von kurzlebigen Wirkstoffen in einem lebenden Organismus am gewünschten Wirkort und über eine gewünschte Zeit auslösen läßt.

Diese Aufgabe löst erfindungsgemäß ein Verfahren mit den Merkmalen des Anspruches 1. Dadurch, daß mittels eines Renaturierungsvorganges aus zumindest einer vorbestimmten Komponente zusammen mit mindestens einem Teil der homogenen Phase ein Endkomplex gebildet wird und daß der Endkomplex nach mindestens einem Zyklus, bestehend aus einem Denaturierungs- und Renaturierungsvorgang, den Wirkstoffkomplex bildet, ist es möglich, Wirkstoffe mit kurzen Halbwertszeiten für die exogene Anwendung nutzbar zu machen. Jede vorbestimmte Komponente ist dabei ein Wirkstoff, den der erfindungsgemäß hergestellte Wirkstoffkomplex bei seinem späteren Einsatz im Organismus aufweisen soll. Unter einem Renaturierungsvorgang soll allgemein das Entfernen des für den Denaturierungsvorgang vorgesehenen Mittels verstanden werden.

Die derart hergestellten Wirkstoffkomplexe haben also den Vorteil, daß jeder in ihnen enthaltene Wirkstoff erst im Organismus zielgerichtet freigesetzt wird. Dabei kann es sich um einen Wirkstoff handeln. Es können aber auch mehrere Wirkstoffe in dem Komplex vorhanden sein. Jeder Zyklus eines Denaturierungs-Renaturierungsvorganges stellt für sich eine Art Anreicherungsschritt (Konzentration) in Bezug auf den jeweils vorbestimmten Wirkstoff dar. Werden mehrere Zyklen durchgeführt, läßt sich jeder vorbestimmte Wirkstoff im Wirkstoffkomplex derart anreichern, daß er nach dem Einbringen in einen Organismus über eine genau definierte und vorherbestimmbare Zeit wirkt. Dadurch werden auch solche an sich endogenen Wirkstoffe einer exogenen Anwendung zugänglich, die im lebenden Organismus nur in geringen Mengen produziert werden und die in einem für die Anwendung des erfindungsgemäßen Verfahrens erforderlichen Ausgangskomplex unter Umständen nur in Spuren vorhanden sind. Dabei liegt es auch noch im Bereich des erfindungsgemäßen Verfahrens, mehrere als Komponenten im Ausgangskomplex enthaltene Wirkstoffe zur Bildung des Wirkstoffkomplexes voneinander unabhängig in ganz verschiedenen Konzentrationen anzureichern.

Bei einer bevorzugten Ausführungsform des erfindungsgemäßen Verfahrens wird jede vorbestimmte Komponente aus einem Teil der homogenen Phase abgereichert oder angereichert oder isoliert. Dabei kann aus der homogenen Phase eine beliebige Anzahl von Teilen gebildet werden.

Vorzugsweise wird die homogene Phase in zwei Teile aufgeteilt, jede vorbestimmte Komponente aus dem einen Teil der homogenen Phase abgereichert oder angereichert oder isoliert und mit dem anderen Teil der homogenen Phase vereint.

Bei einer weiteren bevorzugten Ausführungsform des erfindungsgemäßen Verfahrens wird dem Wirkstoffkomplex zumindest eine weitere, im Ausgangskomplex nicht enthaltene Komponente zugesetzt. Dadurch ergibt sich die Möglichkeit, endogen produzierte Wirkstoffe so mit weiteren Wirkstoffen, die natürlicher oder synthetischer Art sein können, zu kombinieren, daß sich durch die Anwendung des erfindungsgemäßen Wirkstoffkomplexes völlig neuartige, vorherbestimmbare Wirkungen erzielen lassen.

Der bei der Anwendung des erfindungsgemäßen Verfahrens verwendete Ausgangskomplex kann durch Probeentnahme aus tierischen oder humanen Zellen, Geweben oder Organen gebildet werden. Er kann aber auch aus mikrobiellen oder pflanzlichen Zellkulturen oder Organismen gebildet oder synthetisch hergestellt werden.

Weiterhin ist es bei der Anwendung des erfindungsgemäßen Verfahrens vorgesehen, die Denaturierung der Ausgangskomplexe reversibel durchzuführen, so daß keine irreversiblen Veränderungen am Ausgangskomplex oder einer seiner Komponenten auftreten, was den Renaturierungsvorgang erleichtert.

Vorzugsweise können bei der Denaturierung Säuren, Salze, chaotrope Substanzen, wie beispielsweise Harnstoff, Detergentien etc. eingesetzt werden. Die Renaturierung erfolgt erfindungsgemäß durch Entfernung der Denaturierungsmittel, wobei physikalische, chemische oder biologische Verfahren angewendet werden können.

Durch Anwendung des erfindungsgemäßen Verfahrens ist es des weiteren möglich, einen Wirkstoffkomplex bereitzustellen für die Herstellung von biologischen Teilen, insbesondere von Organen für Lebewesen. Ein dahingehender Wirkstoffkomplex ist in der DE-OS 39 36 568 beschrieben. Der Wirkstoffkomplex weist mindestens vier voneinander verschiedene Komponenten (Strukturkomponente, lösliche Rekrutierungskomponente, unlösliche Adhäsionskomponente, Wachstums- und Maturationskomponente) auf, die stofflich nicht miteinander identisch sind. Bringt man Fibronectin zur Bildung der Rekrutierungskomponente in Lösung, muß die Adhäsionskomponente aus einem anderen, von dem Fibronectin verschiedenen Stoff gebildet sein, der nichtlöslich ist. Dasselbe gilt im umgekehrten Fall, sofern man Fibronectin in unlöslicher Form für die Adhäsionskomponente einsetzt. Die angesprochenen, voneinander unterschiedlichen Komponenten sind auf Zielzellen abgestimmt und dienen dem Aufbau der biologischen Teile aus körpereigenen Zellen.

Biologische Teile nehmen für ihre Funktionsleistungen einen gewissen Raum ein. Nicht selten ist ihre Funktion an eine bestimmte Geometrie gebunden, und die biologischen Teile weisen bestimmte Grenzen auf, innerhalb derer sie ihre Funktion erfüllen. Dies gilt in gleicher Weise für die mittels des erfindungsgemäßen Wirkstoffkomplexes hergestellten biologischen Teile. Der für die Herstellung verwendete Wirkstoffkomplex erfüllt diese Funktion mit Hilfe einer Strukturkomponente, die einerseits die Platzhalterfunktion ausübt und es andererseits erlaubt, eine geometrische Form vorzugeben, innerhalb derer das hergestellte biologische Teil seine Funktion erfüllt.

Bei einer bevorzugten Ausführungsform der Strukturkomponente des Wirkstoffkomplexes handelt es sich vorwiegend um eine makromolekulare dreidimensionale Matrix, die zusammen mit Wasser und Salz in der Form eines Gels unterschiedlichen Quellungszustandes vorliegen kann. So kommen z.B. Proteoglycangele als Matrix in Frage. Auch ein Netzwerk von Fasern, wie z.B. verschiedene Arten von Kollagenen oder Elastin können die Strukturkomponente bilden. Ebenso eignen sich Kombinationen von Gelen mit eingelagerten Fasern in Sinne von Verbundwerkstoffen. Für die unterschiedlichen Anwendungen zur Herstellung biologischer Teile wird die Strukturkomponente unterschiedlich konfektioniert, um beispielsweise als Vlies, als Gelkörper oder Flüssiggel schneidbar, fräsbar, plastisch verformbar oder gießbar anwendbar zu sein.

Die Strukturkomponente wird an die Erfordernisse des herzustellenden biologischen Teils angepaßt, da eine gewisse Spezifität zwischen den zellulären und den extrazellulären Anteilen biologischer Teile besteht. Quellen für die Herstellung der Strukturkomponente sind daher vorwiegend die extrazellulären Materialien verschiedener Gewebe oder Organe, d.h. z.B. für die Herstellung von Haut werden für die Produktion der Strukturkomponente kutane Proteoglycane und Faserproteine eingesetzt, für die Herstellung der Milz die milzspezifischen Proteoglycane und Faserproteine, für die Herstellung von Knochen knochenspezifische Proteoglycane und Faserproteine etc.. Die Strukturkomponente kann auch metallische, keramische, glasartige, polymere oder fetthaltige Trägermaterialien aufweisen, mit deren Hilfe die geometrischen, mechanischen, chemischen oder sonstigen Eigenschaften der Strukturkomponente modifizierbar sind. Dabei kann das Trägermaterial zusammen mit der Strukturkomponente in solider, poröser, membranöser, micellarer, viskoser oder flüssiger Form vorliegen, je nach den Anforderungen, die sich für die Herstellung des biologischen Teils und seine spätere Funktion ergeben.

Bei einer weiteren bevorzugten Ausführungsform des erfindungsgemäßen Wirkstoffkomplexes entfaltet dieser seine Wirkung zur Herstellung der biologischen Teile im wesentlichen nur temporär, d.h. der Wirkstoffkomplex ist derart ausgebildet, daß er zeitlich steuerbar, abbaubar und nach der Herstellung des biologischen Teils nicht mehr vorhanden ist. Die Abbaugeschwindigkeit des Wirkstoffkomplexes ist dabei durch unterschiedliche Quervernetzungen der polymeren Matrix und/oder den Zusatz von (Enzym)inhibitoren und/oder immunsuppressiven und/oder entzündungshemmenden Stoffen vorgebbar. Bei den hier angesprochenen Inhibitoren kann es sich um niedermolekulare Verbindungen handeln, die das aktive Zentrum des abbauenden Enzyms besetzen, es kann sich aber auch um Komplexbildner handeln, die einen essentiellen Kofaktor des Enzyms an sich bilden oder um neutralisierende Antikörper. Weitere Inhibitionsmechanismen sind möglich. Als entzündungshemmende und/oder immunsuppressive Zusätze sind hier verwendbar: Hemmstoffe der Phospholipase, wie beispielsweise Steroide, Hemmstoffe der Cyclooxygenase, wie beispielsweise Indomethacin, Hemmstoffe der Lipoxygenase, wie beispielsweise Nordihydroguaretsäure, Immunsuppressiva vom Typ der Cyclosporine und/oder vom Typ des Antithymocyten-Globulins etc..

Zur Herstellung biologischer Teile sind lebende Zellen der gewünschten Art im Bereich der Strukturkomponente anzusammeln. Hierfür enthält die Strukturkomponente des Wirkstoffkomplexes einen oder mehrere Rekrutierungskomponenten, mit deren Hilfe die gewünschten Zellen zu gerichteter Bewegung angeregt werden. Als Rekrutierungskomponente(n) sind Chemotaktika (Chemotaxine) geeignet.

Die jeweils geeigneten Chemotaktika sind für eine Vielzahl von Zellen beschrieben worden und lassen sich aus humanen, animalischen, pflanzlichen oder mikrobiellen Quellen isolieren oder auch durch chemische Synthese oder biotechnische Verfahren herstellen. Wird die außerhalb des Körpers des Lebewesens hergestellte Strukturkomponente mit deren Rekrutierungskomponente(n) in einen Organismus eingebracht und/oder außerhalb desselben mit Zielzellen in Verbindung gebracht, so baut sie einen Konzentrationsgradienten auf, an dem sich die Zielzellen orientieren, wobei die jeweilige Rekrutierungskomponente mit den spezifischen Erkennungsstrukturen auf den Zielzellen, die auch als Receptoren bezeichnet werden, in Wechselwirkung tritt. Für den Fall, daß das herzustellende biologische Teil aus mehreren Zellsorten zusammengesetzt ist, enthält die Strukturkomponente entsprechend der Anzahl der Zellarten mehrere Rekrutierungskomponenten in Form von Chemotaktika.

Die Spezifität der jeweiligen Rekrutierungskomponente für die verschiedenen Zielzellen sowie das Ausmaß der chemotaktischen Aktivitäten wird anhand von Untersuchungen ermittelt, bei denen die gerichtete Wanderung der gewünschten Zellen durch definierte Filterporen unter der Wirkung eines Gradienten des Chemotaktikums in einer Kammer gemessen wird. Mittels derartiger Untersuchungstechniken ist das Wirkstoffsystem hinsichtlich seiner jeweiligen Rekrutierungskomponente biologisch standardisierbar, was für die industriellen Herstellung des Wirkstoffkomplexes von Bedeutung ist.

Als Chemotaktika dienen beispielsweise Peptide wie N-F-Met-Leu-Phe und/oder beispielsweise Metabolite der Arachidonsäure, wie Leukotriene, mit deren Hilfe bestimmte Zellen aus dem Blut bzw. Phagocyten anlockbar sind. Proteine, wie beispielsweise ein Mesenchymzellen anlockendes Protein, wirken speziell auf Bindegewebszellen chemotaktisch.

Neben der Spezifität der jeweiligen Rekrutierungskomponente für die gewünschte Zielzelle und dem Ausmaß der chemotaktischen Aktivität ist auch die Zeitdauer, über die sich der chemotaktischen Konzentrationsgradient aufbaut und erhält, eine zu beachtende Größe. Die Anpassung dieser Kinetik an die Erfordernisse zur Herstellung der biologischen Teile wird bei dem erfindungsgemäßen Wirkstoffkomplex durch eine steuerbare Freisetzung der jeweiligen Rekrutierungskomponente aus der Strukturkomponente erreicht. Hierbei spielt nun die Abbaugeschwindigkeit selbst eine Rolle sowie die Art der Verbindung zwischen Strukturkomponente und der jeweiligen Rekrutierungskomponente, z.B. ob es sich um eine kovalente oder um eine assoziative Bindung handelt. Bei kovalenter Bindung wird ein langsamerer Aufbau und eine längere Erhaltung des chemotaktischen Gradienten erreicht als bei einer lediglich assoziativen Bindung durch ionische Kräfte oder Wasserstoffbrückenbindung. Die Rekrutierung der Zellen zur Herstellung des biologischen Teils verläuft aber meist rascher als der Abbau der Strukturkomponente, da die eingewanderten Zellen ganz wesentlich zum Abbau des Proteoglycan/Collagen Werkstoffes beitragen.

Für die Herstellung der biologischen Teile sind nach der Einwanderung der Zellen in die Strukturkomponente diese am Ort derselben zu fixieren, um ihre Auswanderung in die Umgebung zu verhindern und eine stabile Architektur des hergestellten biologischen Teils zu gewährleisten. Hierfür enthält der Wirkstoffkomplex eine oder mehrere Adhäsionskomponenten, mittels der oder derer die eingewanderten Zellen am Ort der Strukturkomponente fixierbar sind. Dabei "verankert" sich die Adhäsionskomponente einerseits an den die biologischen Teile aufbauenden Zellen und andererseits am makromolekularen Netzwerk der Strukturkomponente. Derartige Adhäsine sind bekannt und weisen eine gewisse Verankerungsspezifität auf. Als Beispiele seien Eiweißkörper vom Typ des Fibronectins oder des Laminins genannt, mit deren Hilfe beispielsweise Bindegewebszellen bzw. Epithelzellen an der Strukturkomponente verankerbar sind. Zahlreiche weitere Adhäsionsfaktoren unterschiedlicher Spezifität sind verfügbar und kommen je nach dem herzustellenden biologischen Teil bei dem erfindungsgemäßen Wirkstoffkomplex zum Einsatz. Hierzu gehören unter anderem die Zell-Adhäsions-Moleküle L-CAM, N-CAM, die Matrix-Adhäsions-Moleküle Cytotactin, Tenascin, Laminin, Fibronectin, Collagen Typ IV,V,VII, sowie synthetische Peptide, die Teilsequenzen verschiedener Adhäsine darstellen und Transmembran-Verbindungsproteine, wie beispielsweise Integrin.

Um bei der Herstellung der biologischen Teile die Spezifität der Anheftung der gewünschten Zellen an die Strukturkomponente zu erhöhen, können Antikörper gegen unerwünschte Adhäsionskomponenten eingesetzt werden. Die biologische Aktivität der Adhäsionskomponenten kann in Adhäsionstests verschiedener Art (z.B. mittels Zentrifugalkräften etc.) gemessen werden und ist damit für den gesamten Wirkstoffkomplex standardisierbar.

Häufig sind die zur Herstellung des biologischen Teils im Bereich der Strukturkomponente chemotaktisch angelockten und durch die geeigneten Adhäsine fixierten Zellen von ihrer Zahl her nicht ausreichend, um das biologische Teil zu konstituieren. Außerdem befinden sich die für diese Prozesse in einem Organismus verfügbaren mobilen Zellen meist nicht in einem hinreichend ausgereiften Zustand, um alle Funktionen eines biologischen Teils zu erfüllen. Sie stellen vielmehr häufig Vorläufer- oder Stammzellen dar, aus denen sich die funktionsfähigen, reifen Zellen des herzustellenden biologischen Teils erst entwickeln müssen. Hierfür weist der erfindungsgemäße Wirkstoffkomplex mindestens eine Wachstums- und/oder Maturationskomponente auf, vorzugsweise in Form eines oder mehrerer Cytokine, unter dessen bzw. deren Einwirkung die Zahl der eingewanderten Zellen vermehrt wird und andererseits eine Ausreifung der Zellen erfolgt.

Cytokine sind Stoffe unterschiedlicher chemischer Struktur, die dadurch gekennzeichnet sind, daß sie mit Zellen in Wechselwirkung treten und deren Teilungs- und Wachstumsverhalten ebenso wie ihre Ausreifung und Biosyntheseleitung beeinflussen. Cytokine haben damit eine hormonähnliche Wirkung, entfalten diese aber im Gegensatz zu den Hormonen nicht in Fernwirkung, sondern in eher lokalisierten Bereichen, was von Vorteil ist bei der Herstellung biologischer Teile, da es sich hierbei ebenfalls um einen lokalisierten Prozeß handelt.

Eine Vielzahl verschiedener Cytokine unterschiedlicher Spezifität ist dem Fachmann bekannt. Diese sind zur Beeinflussung von Zellwachstum, Differenzierung und Maturation (Ausreifung) sowie zur Beeinflussung des Stoffwechsels der eingewanderten Zellen als weitere Komponente im erfindungsgemäßen Wirkstoffsystem einsetzbar. Die Spezifität der Cytokine für bestimmte Zellen ist durch die Anwesenheit der entsprechenden Receptoren auf den Zielzellen determiniert, wobei die Interaktion eines Cytokins mit dem Receptor die zellulären Folgeprozesse auslöst. Bei den hier angesprochenen Receptoren auf den Zielzellen handelt es sich um Membranproteine, die mit dem eingesetzten Chemotaktikum in Wechselwirkung treten, es binden und ins Zellinnere einschleusen. Durch ein Recycling der Rezeptoren stehen sie immer wieder für die Bindung des Chemotaktikum zur Verfügung.

Analog ist es mit den Receptoren für die jeweils eingesetzten Cytokine, so daß es sich nur um eine andere Spezifität handelt bei analogem Mechanismus der Wechselwirkung. Während die Bindung des Chemotaktikums zu gerichteter Bewegung der Zielzellen führt, resultiert die Bindung der Cytokine an den entsprechenden Receptor der Zielzellen in Wachstum und/oder Differenzierung. Vielfach sind die Receptoren molekular noch nicht charakterisiert, so daß sie nur an Hand ihrer Spezifität für den betreffenden Liganden (Chemotaktikum, Cytokin etc.) erkannt werden.

Dabei ist zu berücksichtigen, daß nicht selten stimulierende oder hemmende Folgeprozesse an den Zellen ausgelöst werden können, je nach der Spezifität von eingesetztem Cytokin und Zielzellen. Der für die Herstellung biologischer Teile gewünschte zelluläre Folgeprozeß der Cytokin-Wechselwirkung ist meist an eine duale Signalvermittlung gebunden, so daß in vorteilhafter Weise mindestens zwei Cytokine bei dem erfindungsgemäßen Wirkstoffkomplex verwendet sind, um Wachstum und Differenzierung zu erreichen. Viele Zellen produzieren nach der Interaktion mit einem Cytokin weitere Cytokine und setzen diese frei, wobei sie sich damit selbst stimulieren oder hemmen können (sogenannter autokriner Mechanismus). Nicht selten ändert sich auch mit einzelnen Differenzierungsschritten die Spezifität der Zellen für bestimmte Cytokine, so daß keine Interaktion mehr zustande kommt oder auch die Wechselwirkung von einem stimulierenden in einen hemmenden zellulären Folgeprozeß umschlagen kann. Die Eigenschaften einer Vielzahl von Cytokinen ist bekannt, so daß die Cytokinwirkung im Wirkstoffsystem ebenfalls standardisierbar ist.

Beispiele für Cytokine sind etwa bei der Herstellung von Blut die Kolonie stimulierenden Faktoren, bei der Herstellung von Bindegewebe der Fibroblasten Wachstumsfaktor, bei der Herstellung von Haut der epidermale Wachstumsfaktor, bei der Herstellung von Knorpel der Knorpel induzierende Faktor, bei der Herstellung der Milz oder der Lymphknoten der Lymphocyten aktivierende Faktor sowie Milzpeptide, für die Herstellung von Thymus der T-Zellen Wachstumsfaktor sowie Thymuspeptide, für die Herstellung von Knochen der Knochen-Wachstumsfaktor sowie der transformierende Wachstumsfaktor, für die Herstellung von Blutgefäßen der Angiogenese-Faktor. Ferner finden noch die folgenden Cytokine Verwendung: Interleukine, Insulin ähnliche Wachstumsfaktoren, Tumor Nekrose Faktor, Prostaglandine, Leukotriene, transformierende Wachstumsfaktoren, von Thrombozyten abstammender Wachstumsfaktor, Interferone sowie von Endothelzellen abstammender Wachstumsfaktor.

Da biologische Teile vielfach aus mehreren Zelltypen zusammengesetzt sind, können Kombinationen yorkommen. So ist z.B. die Entstehung von Blutgefäßen für die Versorgung des hergestellten biologischen Teils wichtig, so daß eine beschleunigte Gefäßentstehung durch Zusatz des Angiogenese-Faktors als Cytokinkomponente des Wirkstoffsystems in Frage kommt. Ähnlich kann die beschleunigte Bildung von Nervenverbindungen wichtig sein, was sich durch einen entsprechenden Einsatz von zusätzlichen Cytokinen im Wirkstoffkomplex verwirklichen läßt.

Im folgenden wird die Erfindung anhand eines Ausführungsbeispieles näher erläutert.

### 1. Ausführungsbeispiel:

### Herstellung eines knochenbildenden Wirkstoffkomplexes aus Rinderknochen.

Rinderknochen werden gereinigt, gemahlen und entkalkt. Die auf diese Weise erhaltene Knochenmatrix stellt den Ausgangskomplex für die Anwendung des erfindungsgemäßen Verfahrens dar. Die Knochenmatrix wird mit einer Lösung eines geeigneten Denaturierungsmittels, beispielsweise Harnstoff, versetzt und bei 10°C 24 Stunden gerührt. Als Denaturierungsmittel kommen grundsätzlich Redox-Reaktionen, enzymkatalysierte Umwandlungen, Reaktionen mit chaotropen Salzen, Änderungen von pH, Temperatur oder Ionenstärke, mechanische Einwirkungen, Stoffe mit oberflächenaktiven Eigenschaften (Detergentien) in Frage. Die homogen in Lösung befindlichen Anteile der Knochenmatrix werden abgetrennt. Dies kann z.B. durch Filtration oder Zentrifugation geschehen. Der in homogener Phase befindliche Anteil wird im Volumenverhältnis 1 : 1 geteilt. Mit dem einen Teil der so aufgeteilten denaturierten homogenen Phase wird eine Fraktionierung durchgeführt, die z.B. chromatographisch, elektrophoretisch, durch Ultrafiltration oder durch Dialyse erfolgen kann. Die so erhaltene gewünschte Fraktion wird nun erfindungsgemäß dem zweiten Teil der denaturierten, homogenen Phase zugesetzt und gemeinsam mit dieser renaturiert. Die Renaturierung erfolgt durch Entfernung des Denaturierungsmittels, z.B. durch Filtration. Allgemein können hier verschiedene physikalische, chemische oder biologische Verfahren in Frage kommen. Der so erhaltene Endkomplex ist der für die Knochenbildung geeignete Wirkstoffkomplex.

### 2. Ausführungsbeispiel:

10 kg biologisches Material (z.B. Bindegewebe) werden mit 80 Litern einer wässrigen Lösung eines niedermolekularen Denaturierungsmittels (z.B. 6 molarem Harnstoff) versetzt und bei Zimmertemperatur 72 Stunden gerührt.

Die gelösten Anteile werden vom unlöslichen Rückstand durch ein geeignetes Trennverfahren, beispielsweise durch Zentrifugieren, abgetrennt. Der denaturierte Überstand wird geteilt, beispielsweise im Volumenverhältnis 1 : 1. Aus Teil 1 werden die niedermolekularen Wirkstofffraktionen bis zu einer gewünschten Molmasse, beispielsweise 30000 Dalton, angereichert und der hochmolekulare Substratanteil entfernt, beispielsweise mittels Filtration an einem geeigneten Ultrafilter in einer Hohlfaser-Filterpatrone.

Die derart angereicherte und abgetrennte Wirkstofffraktion wird nun nicht wie im Stand der Technik bekannt auf den Wirkstoff hin aufgearbeitet, sondern mit Teil 2 des denaturierten Überstandes vereinigt und der Renaturierung in Gegenwart des hochmolekularen Substrates zugeführt. Die Renaturierung erfolgt durch Entfernung des denaturierenden Agens, beispielsweise Harnstoff. Hierbei kommt es zur Bildung des Wirkstoffkomplexes, indem sich das Substrat mit den Wirkstoffen verbindet und die Renaturierung des Substrates die Renaturierung der Wirkstoffe begünstigt. Durch Wiederholung des Denaturierungs-Renaturierungs-Zyklus kann das Substrat mit Wirkstoffen gesättigt werden, bis der für die gewünschten biologischen Wirkungen optimierte Wirkstoffkomplex hergestellt ist. Zur Reindarstellung des Wirkstoffkomplexes kann eine Lyophilisation verwendet werden.

Damit wird die Herstellung maßgeschneiderter Wirkstoffkomplexe für unterschiedlichste Anwendungen möglich, indem nach diesem Verfahren beispielsweise strukturelle, chemotaktische, adhäsive, proliferative, differenzierende Eigenschaften im erfindungsgemäßen Wirkstoffkomplex enthalten sind.

Andere Eigenschaften können z.B. die Immunogenität, die Toxizität usw. betreffen und lassen sich im Wirkstoffkomplex durch das erfindungsgemäße Herstellungsverfahren gezielt steuern.

Weitere Anwendungsgebiete für nach dem erfindungsgemäßen Verfahren hergestellte Wirkstoffkomplexe liegen im Bereich der Modifikation von Grenzflächen zur Steigerung der Biokompatibilität oder zur Erzielung bestimmter Wechselwirkungen mit Zellen. Beispielsweise bei der Kultivierung von Zellen für biotechnologische oder für Forschungszwecke sind technische Beschichtungen mit derartigen Wirkstoffkomplexen von Bedeutung. Auch bei diagnostischen oder therapeutischen Systemen, wie z.B. Dialysatoren, Oxygenatoren extrakorporal oder bei künstlichen Organen intrakorporal kommen Anwendungen in Frage.

Alle in der vorstehenden Beschreibung erwähnten Merkmale sind als weitere Ausgestaltungen Bestandteile der Erfindung, auch wenn sie nicht besonders hervorgehoben und insbesondere nicht in den Ansprüchen erwähnt sind.

## Patentansprüche (Patentansprüche für folgende(n) Vertragsstaat(en): AT, BE, CH, DE, DK, FR, GB, IT, LI, LU, MC, NL, SE)

1. Verfahren zur Herstellung von Wirkstoffkomplexen aus mindestens einem Ausgangskomplex, der Komponenten aufweist, bei dem der Ausgangskomplex mittels eines Denaturierungsvorganges zumindest teilweise und unter Zerlegung in seine Komponenten in eine homogene Phase gebracht wird, die zumindest teilweise die Komponenten mit zumindest teilweise geänderter Struktur aufweist, **dadurch gekennzeichnet,**
- **daß** die homogene Phase in zumindest zwei Teile aufgeteilt wird,
- **daß** mittels eines Renaturierungsvorganges aus zumindest einer vorbestimmten Komponente zumindest eines der Teile zusammen mit dem oder den anderen Teil(en) der homogenen Phase ein Endkomplex gebildet wird, wobei jede vorbestimmte Komponente aus dem zumindest einen Teil der homogenen Phase abgereichert oder angereichert wird, und
- **daß** der Endkomplex nach mindestens einem Zyklus, bestehend aus einem Denaturierungs- und Renaturierungsvorgang, den Wirkstoffkomplex bildet.

2. Verfahren nach Anspruch 1, **dadurch gekennzeichnet, daß** die homogene Phase in zwei Teile aufgeteilt wird, daß jede vorbestimmte Komponente aus dem einen Teil der homogenen Phase abgereichert oder angereichert und mit dem anderen Teil der homogenen Phase vereint wird.

3. Verfahren nach Anspruch 1 oder 2, **dadurch gekennzeichnet, daß** dem Wirkstoffkomplex zumindest eine weitere, im Ausgangskomplex nicht enthaltene Komponente zugesetzt wird.

4. Verfahren nach einem der Ansprüche 1 bis 3, **dadurch gekennzeichnet, daß** der Ausgangskomplex aus Zellen, Geweben oder Organen gebildet wird, die tierischen und/oder humanen Ursprungs sind.

5. Verfahren nach einem der Ansprüche 1 bis 3, **dadurch gekennzeichnet, daß** der Ausgangskomplex aus mikrobiellen oder pflanzlichen Zellkulturen oder Organismen gebildet wird.

6. Verfahren nach einem der Ansprüche 1 bis 3, **dadurch gekennzeichnet, daß** der Ausgangskomplex synthetisch hergestellt wird.

7. Verfahren nach einem der Ansprüche 1 bis 6, **dadurch gekennzeichnet, daß** der Denaturierungsvorgang reversibel durchgeführt wird.

8. Verfahren nach einem der Ansprüche 1 bis 7, **dadurch gekennzeichnet, daß** für den Denaturierungsvorgang Säuren, Salze, chaotrope Substanzen, Detergentien eingesetzt werden.

9. Verfahren nach einem der Ansprüche 1 bis 8, **dadurch gekennzeichnet, daß** der Renaturierungsvorgang durch Entfernung eines für den Denaturierungsvorgang vorgesehenen Mittels erfolgt.

10. Wirkstoffkomplex, erhältlich nach dem Verfahren nach einem der Ansprüche 1 bis 9, für die Herstellung von biologischen Teilen, insbesondere von Organen für Lebewesen, **dadurch gekennzeichnet, daß** er
- eine Strukturkomponente,
- mindestens eine Rekrutierungskomponente,
- mindestens eine Adhäsionskomponente und
- mindestens eine Wachstums- und/oder Maturationskomponente, vorzugsweise in Form mindestens eines Cytokins,
aufweist.

11. Wirkstoffkomplex nach Anspruch 10, **dadurch gekennzeichnet, daß** die Strukturkomponente ein makromolekulares Gel und/oder Netzwerk, vorzugsweise aus Proteoglycanen und Kollagenen, ist.

12. Wirkstoffkomplex nach Anspruch 10 oder 11, **dadurch gekennzeichnet, daß** die Strukturkomponente metallische und/oder keramische und/oder glasartige und/oder polymere und/oder fetthaltige Trägermaterialien in solider und/oder poröser und/oder membranöser und/oder micellarer und/oder plastisch verformbarer und/oder viskoser und/oder flüssiger Form aufweist.

13. Wirkstoffkomplex nach einem der Ansprüche 10 bis 12, **dadurch gekennzeichnet, daß** die jeweilige Rekrutierungskomponente zur Gruppe der chemotaktischen Peptide, Proteine und/oder der Arachidonsäurenmetabolite gehört oder eine andere gleichwirkende chemische Struktur aufweist.

14. Wirkstoffkomplex nach einem der Ansprüche 10 bis 13, **dadurch gekennzeichnet, daß** die jeweilige Adhäsionskomponente ein Protein vom Typ des Fibronectin, Tenascin, Cytotactin, Laminin, Chondronectin, Collagen Typ IV,V,VII, N-CAM, L-CAM oder Integrin, eine Kombination dieser Proteine oder ein anderes gleichwirkendes Adhäsin ist.

15. Wirkstoffkomplex nach einem der Ansprüche 10 bis 14, **dadurch gekennzeichnet, daß** die jeweilige Wachstums- und/oder Maturationskomponente zur Gruppe der Kolonie stimulierenden Faktoren (CSF), der Interleukine (IL), der Insulin ähnlichen Wachstumsfaktoren (IGF), der Prostaglandine (PG), der Leukotriene (LT), der transformierenden Wachstumsfaktoren (TGF), der Fibroblasten Wachstumsfaktoren (FGF), der Interferone (IFN), der epidermalen Wachstumsfaktoren (EGF), der von Knochen abstammenden Wachstumsfaktoren (BDGF), der von Endothelien abstammenden Wachstumsfaktoren (EDGF), der von Trombocyten abstammenden Wachstumsfaktoren (PDGF) gehört, eine Kombination derartiger Faktoren oder ein anderes gleichwirkendes Cytokin ist.

16. Wirkstoffkomplex nach einem der Ansprüche 10 bis 15, **dadurch gekennzeichnet, daß** die jeweilige Wachstums- und/ oder Maturationskomponente Faktoren zum beschleunigten Einwachsen von Blutgefäßen, wie Angioginese-Faktoren und/oder von Nerven, wie Nervenwachstums-Faktoren, in das herzustellende biologische Teil aufweist.

17. Verwendung des Wirkstoffkomplexes nach einem der Ansprüche 10 bis 16 zur Herstellung eines biologischen Teiles zur Behandlung von Krankheiten, die durch das Fehlen des biologischen Teiles oder dessen Fehlfunktion bedingt sind.

## Patentansprüche (Patentansprüche für folgende(n) Vertragsstaat(en): ES, GR)

1. Verfahren zur Herstellung von Wirkstoffkomplexen aus mindestens einem Ausgangskomplex, der Komponenten aufweist, bei dem der Ausgangskomplex mittels eines Denaturierungsvorganges zumindest teilweise und unter Zerlegung in seine Komponenten in eine homogene Phase gebracht wird, die zumindest teilweise die Komponenten mit zumindest teilweise geänderter Struktur aufweist, **dadurch gekennzeichnet,**
- **daß** die homogene Phase in zumindest zwei Teile aufgeteilt wird,
- **daß** mittels eines Renaturierungsvorganges aus zumindest einer vorbestimmten Komponente zumindest eines der Teile zusammen mit dem oder den anderen Teil(en) der homogenen Phase ein Endkomplex gebildet wird, wobei jede vorbestimmte Komponente aus dem zumindest einen Teil der homogenen Phase abgereichert oder angereichert wird, und
- daß der Endkomplex nach mindestens einem Zyklus, bestehend aus einem Denaturierungs- und Renaturierungsvorgang, den Wirkstoffkomplex bildet.

2. Verfahren nach Anspruch 1, **dadurch gekennzeichnet, daß** die homogene Phase in zwei Teile aufgeteilt wird, daß jede vorherbestimmte Komponente aus dem einen Teil der homogenen Phase abgereichert oder angereichert und mit dem anderen Teil der homogenen Phase vereint wird.

3. Verfahren nach Anspruch 1 oder 2, **dadurch gekennzeichnet, daß** dem Wirkstoffkomplex zumindest eine weitere, im Ausgangskomplex nicht enthaltene Komponente zugesetzt wird.

4. Verfahren nach einem der Ansprüche 1 bis 3, **dadurch gekennzeichnet, daß** der Ausgangskomplex aus Zellen, Geweben oder Organen gebildet wird, die tierischen und/oder humanen Ursprungs sind.

5. Verfahren nach einem der Ansprüche 1 bis 3, **dadurch gekennzeichnet, daß** der Ausgangskomplex aus mikrobiellen oder pflanzlichen Zellkulturen oder Organismen gebildet wird.

6. Verfahren nach einem der Ansprüche 1 bis 3, **dadurch gekennzeichnet, daß** der Ausgangskomplex synthetisch hergestellt wird.

7. Verfahren nach einem der Ansprüche 1 bis 6, **dadurch gekennzeichnet, daß** der Denaturierungsvorgang reversibel durchgeführt wird.

8. Verfahren nach einem der Ansprüche 1 bis 7, **dadurch gekennzeichnet, daß** für den Denaturierungsvorgang Säuren, Salze, chaotrope Substanzen, Detergentien eingesetzt werden.

9. Verfahren nach einem der Ansprüche 1 bis 8, **dadurch gekennzeichnet, daß** der Renaturierungsvorgang durch Entfernung eines für den Denaturierungsvorgang vorgesehenen Mittels erfolgt.

10. Verfahren nach einem der Ansprüche 1 bis 9, **dadurch gekennzeichnet, daß** ein Wirkstoffkomplex für die Herstellung von biologischen Teilen, insbesondere von Organen für Lebewesen hergestellt wird, der
- eine Strukturkomponente,
- mindestens eine Rekrutierungskomponente,
- mindestens eine Adhäsionskomponente und
- mindestens eine Wachstums- und/oder Maturationskomponente, vorzugsweise in Form mindestens eines Cytokins,
aufweist.

11. Verfahren nach Anspruch 10, **dadurch gekennzeichnet, daß** als Strukturkomponente ein makromolekulares Gel und/oder Netzwerk, vorzugsweise aus Proteoglycanen und Kollagenen, eingesetzt wird.

12. Verfahren nach Anspruch 10 oder 11, **dadurch gekennzeichnet, daß** die Strukturkomponente metallische und/oder keramische und/oder glasartige und/oder polymere und/oder fetthaltige Trägermaterialien in solider und/oder poröser und/oder membranöser und/oder micellarer und/oder plastisch verformbarer und/oder viskoser und/oder flüssiger Form aufweist.

13. Verfahren nach einem der Ansprüche 10 bis 12, **dadurch gekennzeichnet, daß** die jeweilige Rekrutierungskomponente zur Gruppe der chemotaktischen Peptide, Proteine und/oder der Arachidonsäuremetabolite gehört oder eine andere gleichwirkende chemische Struktur aufweist.

14. Verfahren nach einem der Ansprüche 10 bis 13, **dadurch gekennzeichnet, daß** als jeweilige Adhäsionskomponente ein Protein vom Typ des Fibronectin, Tenascin, Cytotactin, Laminin, Chondronectin, Collagen Typ IV, V, VII, N-CAM, L-CAM oder Integrin, eine Kombination dieser Proteine oder ein anderes gleichwirkendes Adhäsin eingesetzt wird.

15. Verfahren nach einem der Ansprüche 10 bis 14, **dadurch gekennzeichnet, daß** die jeweilige Wachstums- und/oder Maturationskomponente ausgewählt wird aus der Gruppe der Kolonie stimulierenden Faktoren (CSF), der Interleukine (IL), der Insulin ähnlichen Wachstumsfaktoren (IGF), der Prostaglandine (PG), der Leukotriene (LT), der transformierenden Wachstumsfaktoren (TGF), der Fibroblasten Wachstumsfaktoren (FGF), der Interferone (IFN), der epidermalen Wachstumsfaktoren (EGF), der von Knochen abstammenden Wachstumsfaktoren (BDGF), der von Endothelien abstammenden Wachstumsfaktoren (EDGF), der von Trombocyten abstammenden Wachstumsfaktoren (PDGF), einer Kombination derartiger Faktoren oder einem anderen gleichwirkenden Cytokin.

16. Verfahren nach einem der Ansprüche 10 bis 15, **dadurch gekennzeichnet, daß** die jeweilige Wachstums- und/oder Maturationskomponente Faktoren zum beschleunigten Einwachsen von Blutgefäßen, wie Angioginese-Faktoren und/oder von Nerven, wie Nervenwachstums-Faktoren, in das herzustellende biologische Teil aufweist.

17. Verfahren nach einem der Ansprüche 10 bis 16, bei dem der gemäß einem der Ansprüche 10 bis 16 hergestellte Wirkstoffkomplex zur Herstellung eines biologischen Teiles zur Behandlung von Krankheiten, die durch das Fehlen des biologischen Teiles oder dessen Fehlfunktion bedingt sind, verwendet wird.

## Claims (Claims for the following Contracting State(s): AT, BE, CH, DE, DK, FR, GB, IT, LI, LU, MC, NL, SE)

1. A process for producing active substance complexes from at least one initial complex having components, in which the initial complex is brought by means of a denaturation process at least partly, and with disintegration into its components, into a homogeneous phase which at least partly has the components with at least partly altered structure, **characterized**
- **in that** the homogeneous phase is divided into at least two parts,
- **in that** a final complex is formed by means of a renaturation process from at least one predetermined component of at least one of the parts together with the other part(s) of the homogeneous phase, each predetermined component being depleted or enriched from the at least one part of the homogeneous phase, and
- **in that** the final complex forms, after at least one cycle comprising a denaturation process and renaturation process, the active substance complex.

2. A process according to claim 1, **characterized in that** the homogeneous phase is divided into two parts, **in that** each predetermined component is depleted or enriched from the one part of the homogeneous phase and combined with the other part of the homogeneous phase.

3. A process according to either of claims 1 and 2, **characterized in that** at least one other component not present in the initial complex is added to the active substance complex.

4. A process according to any one of claims 1 to 3, **characterized in that** the initial complex is formed from cells, tissues or organs of animal and/or human origin.

5. A process according to any one of claims 1 to 3, **characterized in that** the initial complex is formed from microbial or plant cell cultures or organisms.

6. A process according to any one of claims 1 to 3, **characterized in that** the initial complex is produced synthetically.

7. A process according to any one of claims 1 to 6, **characterized in that** the denaturation process is carried out reversibly.

8. A process as claimed in any one of claims 1 to 7, **characterized in that** acids, salts, chaotropic substances, detergents are employed for the denaturation process.

9. A process according to any one of claims 1 to 8, **characterized in that** the renaturation process takes place by removing an agent provided for the denaturation process.

10. An active substance complex obtainable by the process according to any one of claims 1 to 9 for producing biological parts, in particular organs for living creatures, **characterized in that** it has
- a structural component,
- at least one recruiting component,
- at least one adhesion component and
- at least one growth and/or maturation component, preferably in the form of at least one cytokine.

11. An active substance complex according to claim 10, **characterized in that** the structural component is a macromolecular gel and/or network, preferably composed of proteoglycans and collagens.

12. An active substance complex according to either of claims 10 and 11, **characterized in that** the structural component has metallic and/or ceramic and/or glassy and/or polymeric and/or fat-containing carrier materials in solid and/or porous and/or membranous and/or micellar and/or plastically deformable and/or viscous and/or liquid form.

13. An active substance complex according to any one of claims 10 to 12, **characterized in that** the particular recruiting component belongs to the group of chemotactic peptides, proteins and/or arachidonic acid metabolites, or has another chemical structure with the same effect.

14. An active substance complex according to any one of claims 10 to 13, **characterized in that** the particular adhesion component is a protein of the type of fibronectin, tenascin, cytotactin, laminin, chondronectin, collagen type IV, V, VII, N-CAM, L-CAM or integrin, a combination of these proteins or another adhesin with the same effect.

15. An active substance complex according to any one of claims 10 to 14, **characterized in that** the particular growth and/or maturation component belongs to the group of colony stimulating factors (CSF), of interleukins (IL), of insulin-like growth factors (IGF), of prostaglandins (PG), of leucotrienes (LT), of transforming growth factors (TGF), of fibroblast growth factors (FGF), of interferons (IFN), of epidermal growth factors (EGF), of bone-derived growth factors (BDGF), of endothelium-derived growth factors (EDGF), of platelet-derived growth factors (PDGF), a combination of such factors or another cytokine with the same effect.

16. An active substance complex according to any one of claims 10 to 15, **characterized in that** the particular growth and/or maturation component has factors for accelerated growth of blood vessels, such as angiogenesis factors, and/or of nerves, such as nerve growth factors, into the biological part to be produced.

17. The use of the active substance complex according to any one of claims 10 to 16 for producing a biological part for the treatment of disorders caused by the absence of the biological part or dysfunction thereof.

## Claims (Claims for the following Contracting State(s): ES, GR)

1. A process for producing active substance complexes from at least one initial complex having components, in which the initial complex is brought by means of a denaturation process at least partly, and with disintegration into its components, into a homogeneous phase which at least partly has the components with at least partly altered structure, **characterized**
- **in that** the homogeneous phase is divided into at least two parts,
- **in that** a final complex is formed by means of a renaturation process from at least one predetermined component of at least one of the parts together with the other part(s) of the homogeneous phase, each predetermined component being depleted or enriched from the at least one part of the homogeneous phase, and
- **in that** the final complex forms, after at least one cycle comprising a denaturation process and renaturation process, the active substance complex.

2. A process according to claim 1, **characterized in that** the homogeneous phase is divided into two parts, **in that** each predetermined component is depleted or enriched from the one part of the homogeneous phase and combined with the other part of the homogeneous phase.

3. A process according to either of claims 1 and 2, **characterized in that** at least one other component not present in the initial complex is added to the active substance complex.

4. A process according to any one of claims 1 to 3, **characterized in that** the initial complex is formed from cells, tissues or organs of animal and/or human origin.

5. A process according to any one of claims 1 to 3, **characterized in that** the initial complex is formed from microbial or plant cell cultures or organisms.

6. A process according to any one of claims 1 to 3, **characterized in that** the initial complex is produced synthetically.

7. A process according to any one of claims 1 to 6, **characterized in that** the denaturation process is carried out reversibly.

8. A process as claimed in any one of claims 1 to 7, **characterized in that** acids, salts, chaotropic substances, detergents are employed for the denaturation process.

9. A process according to any one of claims 1 to 8, **characterized in that** the renaturation process takes place by removing an agent provided for the denaturation process.

10. A process according to any one of claims 1 to 9, **characterized in that** an active substance complex for producing biological parts, in particular organs for living creatures, which has
- a structural component,
- at least one recruiting component,
- at least one adhesion component and
- at least one growth and/or maturation component, preferably in the form of at least one cytokine,
is produced.

11. A process according to claim 10, **characterized in that** the structural component employed is a macromolecular gel and/or network, preferably composed of proteoglycans and collagens.

12. A process according to either of claims 10 and 11, **characterized in that** the structural component has metallic and/or ceramic and/or glassy and/or polymeric and/or fat-containing carrier materials in solid and/or porous and/or membranous and/or micellar and/or plastically deformable and/or viscous and/or liquid form.

13. A process according to any one of claims 10 to 12, **characterized in that** the particular recruiting component belongs to the group of chemotactic peptides, proteins and/or arachidonic acid metabolites, or has another chemical structure with the same effect.

14. A process according to any one of claims 10 to 13, **characterized in that** the particular adhesion component employed is a protein of the type of fibronectin, tenascin, cytotactin, laminin, chondronectin, collagen type IV, V, VII, N-CAM, L-CAM or integrin, a combination of these proteins or another adhesin with the same effect.

15. A process according to any one of claims 10 to 14, **characterized in that** the particular growth and/or maturation component is selected from the group of colony stimulating factors (CSF), of interleukins (IL), of insulin-like growth factors (IGF), of prostaglandins (PG), of leucotrienes (LT), of transforming growth factors (TGF), of fibroblast growth factors (FGF), of interferons (IFN), of epidermal growth factors (EGF), of bone-derived growth factors (BDGF), of endothelium-derived growth factors (EDGF), of platelet-derived growth factors (PDGF), a combination of such factors or another cytokine with the same effect.

16. A process according to any one of claims 10 to 15, **characterized in that** the particular growth and/or maturation component has factors for accelerated growth of blood vessels, such as angiogenesis factors, and/or of nerves, such as nerve growth factors, into the biological part to be produced.

17. A process according to any one of claims 10 to 16 in which the active substance complex produced according to any one of claims 10 to 16 is used for producing a biological part for the treatment of disorders caused by the absence of the biological part or dysfunction thereof.

## Revendications (Revendications pour l'(les) Etat(s) contractant(s) suivant(s): AT, BE, CH, DE, DK, FR, GB, IT, LI, LU, MC, NL, SE)

1. Procédé pour la préparation de complexes de substances actives à partir d'au moins un complexe de départ comprenant un certain nombre de composants, dans lequel on amène le complexe de départ, dans une étape de dénaturation, en le décomposant en ses composants, au moins partiellement en phase homogène, laquelle phase homogène présente au moins partiellement les composants avec une structure au moins partiellement modifiée, lequel procédé est **caractérisé par le fait**
- **que** la phase homogène est divisée en au moins deux parties,
- **que** l'on forme, dans une étape de renaturation, à partir d'au moins un composant prédéterminé d'au moins une des parties, avec la ou les autres parties de la phase homogène un complexe final, chaque composant prédéterminé étant appauvri ou enrichi à partir de la partie ou des parties de la phase homogène, et
- **que** le complexe final, forme après au moins un cycle constitué d'une étape de dénaturation et d'une étape de renaturation, le complexe de substances actives.

2. Procédé selon la revendication 1, **caractérisé par le fait que** la phase homogène est divisée en deux parties, que chaque composant prédéterminé est appauvri ou enrichi à partir de l'une des parties de la phase homogène et est uni à l'autre partie de la phase homogène.

3. Procédé selon la revendication 1 ou 2, **caractérisé par le fait que** l'on ajoute au complexe de substances actives au moins un composant supplémentaire non présent dans le complexe de départ.

4. Procédé selon une des revendications 1 à 3, **caractérisé par le fait que** le complexe de départ est formé de cellules, de tissus ou d'organes d'origine animale et/ou d'origine humaine.

5. Procédé selon une des revendications 1 à 3, **caractérisé par le fait que** le complexe de départ est formé de cultures de cellules ou d'organismes végétaux ou de microorganismes.

6. Procédé selon une des revendications 1 à 3, **caractérisé par le fait que** le complexe de départ est préparé par synthèse.

7. Procédé selon l'une des revendications 1 à 6, **caractérisé par le fait que** l'étape de dénaturation est réversible.

8. Procédé selon une des revendications 1 à 7, **caractérisé par le fait que** l'on utilise pour l'étape de dénaturation des acides, des sels, des substances chaotropiques ou des agents détergents.

9. Procédé selon une des revendications 1 à 8, **caractérisé par le fait que** l'étape de renaturation se fait par élimination de l'agent utilisé pour l'étape de dénaturation.

10. Complexe de substances actives, susceptible d'être préparé par le procédé selon une des revendications 1 à 9, pour la fabrication d'éléments biologiques, en particulier d'organes d'êtres vivants, **caractérisé par le fait qu'**il comprend
- un composant de structure,
- au moins un composant de recrutement,
- au moins un composant d'adhésion et
- au moins un composant de croissance et/ou de maturation, de préférence sous forme d'une cytokine.

11. Complexe de substances actives selon la revendication 10, **caractérisé par le fait que** l'on utilise comme composant de structure un gel et/ou réseau macromoléculaire, de préférence constitué de protéoglycanes et de collagènes.

12. Complexe de substances actives selon la revendication 10 ou 11, **caractérisé par le fait que** le composant de structure comprend des matériaux support métalliques et/ou céramiques et/ou vitreux et/ou polymériques et/ou gras sous forme solide et/ou poreuse et/ou membranaire et/ou micellaire et/ou plastique et/ou visqueuse et/ou liquide.

13. Complexe de substances actives selon une des revendications 10 à 12, **caractérisé par le fait que** le composant de recrutement appartient au groupe des peptides chimiotactiques, des protéines et/ou des métabolites de l'acide arachidonique ou présente une structure chimique différente ayant une action équivalente.

14. Complexe de substances actives selon une des revendications 10 à 13, **caractérisé par le fait que** l'on utilise, comme composant d'adhésion, une protéine de type fibronectine, ténascine, cytotactine, laminine, chondronectine, collagène de type IV, V, VII, N-CAM, L-CAM ou intégrine, une combinaison de ces protéines ou une autre adhésine ayant une action similaire.

15. Complexe de substances actives selon une des revendications 10 à 14, **caractérisé par le fait que** le composant de croissance et/ou de maturation est choisi dans le groupe formé par les facteurs stimulant la croissance de colonies (CSF), les interleukines (IL), les facteurs de croissance semblables à l'insuline (IGF,) les prostaglandines (PG), les leucotriènes (LT), les facteurs de croissance transformants (TGF), les facteurs de croissance des fibroblastes (FGF), les interférons (IFN), les facteurs de croissance de l'épiderme (EGF), les facteurs de croissance dérivés des tissus osseux (BDGF), les facteurs de croissance dérivés del'endothélium (EDGF), les facteurs de croissance dérivés des thrombocytes (PDGF), une combinaison de ces facteurs ou une autre cytokine ayant une action équivalente.

16. Complexe de substances actives selon une des revendications 10 à 15, **caractérisé par le fait que** le composant de croissance et/ou de maturation contient des facteurs stimulant l'invasion de l'élément biologique par des vaisseaux sanguins, tels que des facteurs d'angiogenèse, et/ou des facteurs stimulant l'invasion de l'élément biologique par des nerfs, tels que les facteurs de croissance des nerfs.

17. Utilisation du complexe de substances actives selon une des revendications 10 à 16, pour fabriquer un élément biologique destiné au traitement de maladies dues à l'absence de l'élément biologique ou au dysfonctionnement de celui-ci.

## Revendications (Revendications pour l'(les) Etat(s) contractant(s) suivant(s): ES, GR)

1. Procédé pour la préparation de complexes de substances actives à partir d'au moins un complexe de départ comprenant un certain nombre de composants, dans lequel on amène le complexe de départ, dans une étape de dénaturation, en le décomposant en ses composants, au moins partiellement en phase homogène, laquelle phase homogène présente au moins partiellement les composants avec une structure au moins partiellement modifiée, lequel procédé est **caractérisé par le fait**
- **que** la phase homogène est divisée en au moins deux parties,
- **que** l'on forme, dans une étape de renaturation, à partir d'au moins un composant prédéterminé d'au moins une des parties, avec la ou les autres parties de la phase homogène un complexe final, chaque composant prédéterminé étant appauvri ou enrichi à partir de la partie ou des parties de la phase homogène, et
- **que** le complexe final, forme après au moins un cycle constitué d'une étape de dénaturation et d'une étape de renaturation, le complexe de substances actives.

2. Procédé selon la revendication 1, **caractérisé par le fait que** la phase homogène est divisée en deux parties, que chaque composant prédéterminé est appauvri ou enrichi à partir de l'une des parties de la phase homogène et est uni à l'autre partie de la phase homogène.

3. Procédé selon la revendication 1 ou 2, **caractérisé par le fait que** l'on ajoute au complexe de substances actives au moins un composant supplémentaire non présent dans le complexe de départ.

4. Procédé selon une des revendications 1 à 3, **caractérisé par le fait que** le complexe de départ est formé de cellules, de tissus ou d'organes d'origine animale et/ou d'origine humaine.

5. Procédé selon une des revendications 1 à 3, **caractérisé par le fait que** le complexe de départ est formé de cultures de cellules ou d'organismes végétaux ou de microorganismes.

6. Procédé selon une des revendications 1 à 3, **caractérisé par le fait que** le complexe de départ est préparé par synthèse.

7. Procédé selon l'une des revendications 1 à 6, **caractérisé par le fait que** l'étape de dénaturation est réversible.

8. Procédé selon une des revendications 1 à 7, **caractérisé par le fait que** l'on utilise pour l'étape de dénaturation des acides, des sels, des substances chaotropiques ou des agents détergents.

9. Procédé selon une des revendications 1 à 8, **caractérisé par le fait que** l'étape de renaturation se fait par élimination de l'agent utilisé pour l'étape de dénaturation.

10. Procédé selon une des revendications 1 à 9, **caractérisé par le fait que** l'on prépare un complexe de substances actives pour la fabrication d'éléments biologiques, en particulier d'organes d'êtres vivants, qui comprend
- un composant de structure.
- au moins un composant de recrutement,
- au moins un composant d'adhésion et
- au moins un composant de croissance et/ou de maturation, de préférence sous forme d'une cytokine.

11. Procédé selon la revendication 10, **caractérisé par le fait que** l'on utilise comme composant de structure un gel et/ou réseau macromoléculaire, de préférence constitué de protéoglycanes et de collagènes.

12. Procédé selon la revendication 10 ou 11, **caractérisé par le fait que** le composant de structure comprend des matériaux support métalliques et/ou céramiques et/ou vitreux et/ou polymériques et/ou gras sous forme solide et/ou poreuse et/ou membranaire et/ou micellaire et/ou plastique et/ou visqueuse et/ou liquide.

13. Procédé selon une des revendications 10 à 12, **caractérisé par le fait que** le composant de recrutement appartient au groupe des peptides chimiotactiques, des protéines et/ou des métabolites de l'acide arachidonique ou présente une structure chimique différente ayant une action équivalente.

14. Procédé selon une des revendications 10 à 13, **caractérisé par le fait que** l'on utilise, comme composant d'adhésion, une protéine de type fibronectine, ténascine, cytotactine, laminine, chondronectine, collagène de type IV, V, VII, N-CAM, L-CAM ou intégrine. une combinaison de ces protéines ou une autre adhésine ayant une action similaire.

15. Procédé selon une des revendications 10 à 14, **caractérisé par le fait que** le composant de croissance et/ou de maturation est choisi dans le groupe formé par les facteurs stimulant la croissance de colonies (CSF), les interleukines (IL), les facteurs de croissance semblables à l'insuline (IGF,) les prostaglandines (PG), les leucotriènes (LT), les facteurs de croissance transformants (TGF), les facteurs de croissance des fibroblastes (FGF), les interférons (IFN), les facteurs de croissance de l'épiderme (EGF), les facteurs de croissance dérivés des tissus osseux (BDGF), les facteurs de croissance dérivés de l'endothélium (EDGF), les facteurs de croissance dérivés des thrombocytes (PDGF), une combinaison de ces facteurs ou une autre cytokine ayant une action équivalente.

16. Procédé selon une des revendications 10 à 15, **caractérisé par le fait que** le composant de croissance et/ou de maturation contient des facteurs stimulant l'invasion de l'élément biologique par des vaisseaux sanguins, tels que des facteurs d'angiogenèse, et/ou des facteurs stimulant l'invasion de l'élément biologique par des nerfs, tels que les facteurs de croissance des nerfs.

17. Procédé selon une des revendications 10 à 16, dans lequel on utilise le complexe de substances actives préparé par un procédé selon une des revendications 10 à 16, pour fabriquer un élément biologique destiné au traitement de maladies dues à l'absence de l'élément biologique ou au dysfonctionnement de celui-ci.
